# EUROPÄISCHE PATENTANMELDUNG

(11) **EP 2 614 794 A1**
(43) Veröffentlichungstag der Anmeldung: **17.07.2013**
(21) Anmeldenummer: 12192570.5
(22) Anmeldetag: 14.11.2012
(51) Int. Cl.: A61F 2/24

(54) **Herzklappenprothese**

(30) Priorität: 11.01.2012 US 201261585273 P
(71) Anmelder: Biotronik AG, 8180 Bülach (CH)
(72) Erfinder: Fargahi, Amir, 8180 Bülach (CH)
(74) Vertreter: Lindner-Vogt, Karin L.

(57) **Zusammenfassung**

Herzklappenprothese aus biologischem Material oder Kunststoff, mit einer Naht (17) aus einem röntgen-sichtbaren Metalldraht, deren Position die Klappenebene kennzeichnet.

## Beschreibung

Die Erfindung betrifft eine Herzklappenprothese aus biologischem Material oder Kunststoff.

Derartige Prothesen sind seit langem bekannt und im klinischen Einsatz. Seit einigen Jahren gibt es auch Ausführungen, die in einen Vaskularstent eingesetzt sind und mit minimal invasiven Operationstechniken eingesetzt werden können; so etwa die Produkte "Sapien" der Firma Edwards Lifesciences Inc. oder "Corevalve" der Firma Medtronic Inc.

Während das Einsetzen einer Herzklappenprothese am offenen Herzen unter direkter Sichtkontrolle des Operateurs erfolgt und dabei insbesondere die Prothese mit korrekter Lage der Herzklappenebene positioniert werden kann, muss die korrekte Positionierung beim minimal invasiven Eingriff allein anhand der Röntgenkontrolle erfolgen. Es hat sich dabei als schwierig herausgestellt, die Lage der Herzklappenebene festzustellen, womit auch die zuverlässig korrekte Positionierung der Prothese problematisch ist.

Der Erfindung liegt daher die Aufgabe der Bereitstellung einer verbesserten Herzklappenprothese zugrunde, welche insbesondere unter Röntgenkontrolle leicht und zuverlässig korrekt positioniert werden kann.

Diese Aufgabe wird durch eine Herzklappenprothese mit den Merkmalen des Anspruchs 1 gelöst. Zweckmäßige Fortbildungen des Erfindungsgedankens sind Gegenstand der abhängigen Ansprüche.

Die Erfindung schließt den Gedanken ein, an der Herzklappenprothese eine röntgensichtbare Markierung vorzunehmen. Sie schließt weiter den Gedanken ein, hierbei das Anbringen von den Aufbau der Prothese verkomplizierenden und/oder möglicherweise die Funktion beeinträchtigenden Röntgenmarkem - insbesondere von an sich bekannten harten, flächigen Markern - zu vermeiden. Aus diesem Grund wird das Vorsehen einer Naht oder ggfs. auch nur eines Knotens aus röntgen-sichtbarem Material (Metalldraht) vorgeschlagen.

Wenn eine in der Prothese ohnehin vorzusehende Naht aus diesem Material gefertigt wird, ändert sich an der Funktion grundsätzlich nichts, und der Herstellungsaufwand steigt nicht wesentlich an. Es ist somit eine Kennzeichnung der Herzklappenebene unter Röntgenkontrolle erreichbar, ohne die Funktionsfähigkeit der Prothese einzuschränken, und dies verbessert die Gebrauchsfähigkeit im klinischen Einsatz und somit auch die Marktperspektive. Weitgehend lässt sich dies auch in einer Ausführung erreichen, bei der die Naht aus dem Metalldraht einen Abschnitt einer aus einem anderen Nahtmaterial gefertigten Naht doppelt.

In aus derzeitiger Sicht bevorzugten Ausführungen weist der Metalldraht mindestens eines der Elemente Gold, Platin, Tantal und Wolfram auf, wobei er insbesondere aus Gold, Platin oder Wolfram besteht oder mit mindestens einem der Elemente Gold, Platin, Wolfram oder Tantal beschichtet ist. Grundsätzlich sind auch andere Metalle als Nahtmaterial oder jedenfalls Bestandteil eines Nahtmaterials einsetzbar, wie etwa Edelstahl oder ein anderes Weißmetall als Platin oder Legierungen aus zwei oder mehreren der genannten Elemente.

In einer weiteren Ausgestaltung der Erfindung ist vorgesehen, dass der Metalldraht einen Durchmesser im Bereich zwischen 20 und 200µm, insbesondere zwischen 50 und 100µm, hat. Bei Einsatz eines röntgen-sichtbaren Nahtmaterials, welches nicht vollständig durch einen Metalldraht gebildet, sondern bei dem ein feiner Metalldraht beispielsweise mit herkömmlichem Nahtmaterial verzwirnt ist, sind Drahtdurchmesser nahe der genannten Untergrenze oder sogar darunter möglich - vorausgesetzt, dass eine hinreichende Sichtbarkeit im Röntgenbild noch gegeben ist. In diesem Zusammenhang sehen weitere Ausgestaltungen der Erfindung vor, dass die Naht aus dem röntgen-sichtbaren Metalldraht als Doppel-, Kapp-, Overlock- oder sonstige optisch auffällige Naht ausgeführt ist.

Grundsätzlich liegt es im Rahmen der Erfindung, eine Naht aus röntgen-sichtbarem Material auszuführen, deren Verlauf (Längserstreckung) in bekannter Weise zur Lage der Klappenebene korrespondiert und diese somit kennzeichnet. Erfindungsgemäß sind aber auch Ausführungen, bei denen die Naht aus dem röntgen-sichtbaren Metalldraht derart platziert ist, dass ein vorbestimmtes Ende hiervon die Position der Klappenebene kennzeichnet.

In einer typischen konstruktiven Ausführung einer z.B. Aorten-Herzklappenprothese mit einem Grundkörper, in den drei Klappensegel mit je einer Naht eingenäht sind, ist an jedem Klappensegel ein Nahtabschnitt aus dem röntgen-sichtbaren Metalldraht vorgesehen, derart, dass die drei Nahtabschnitte gemeinsam die Klappenebene kennzeichnen. Eine alternative Lösung sieht vor, dass bei einer in einen Vaskularstent eingenähten Herzklappenprothese lediglich der Vaskularstent einen Nahtabschnitt aus dem röntgen-sichtbaren Metalldraht aufweist, deren Lage die Klappenebene kennzeichnet. Auch hier sollen Knoten als Nahtabschnitte zu verstehen sein.

Eine weitere Ausführung gemäß der vorliegenden Erfindung stellt eine Mitralklappe dar, in der zwei Klappensegel wie oben beschrieben mit je einer Naht aus einem röntgen-sichtbaren Metalldraht eingenäht sind.

Vorteile und Zweckmäßigkeiten der Erfindung ergeben sich im Übrigen aus der nachfolgenden Beschreibung von Ausführungsbeispielen und -aspekten anhand der Figuren. Von diesen zeigen:
- Fig. 1: eine schematische perspektivische Darstellung einer z.B. Aorten-Herzklappenprothese mit wesentlichen Nähten,
- Fig. 2: eine perspektivische Darstellung einer minimal invasiv implantierbaren Herzklappenprothese und
- Fig. 3: eine Detailansicht eines Verbindungsbereiches zwischen der eigentlichen Herzklappe und dem Vaskularstent bei einer minimal invasiv implantierbaren Herzklappenprothese.

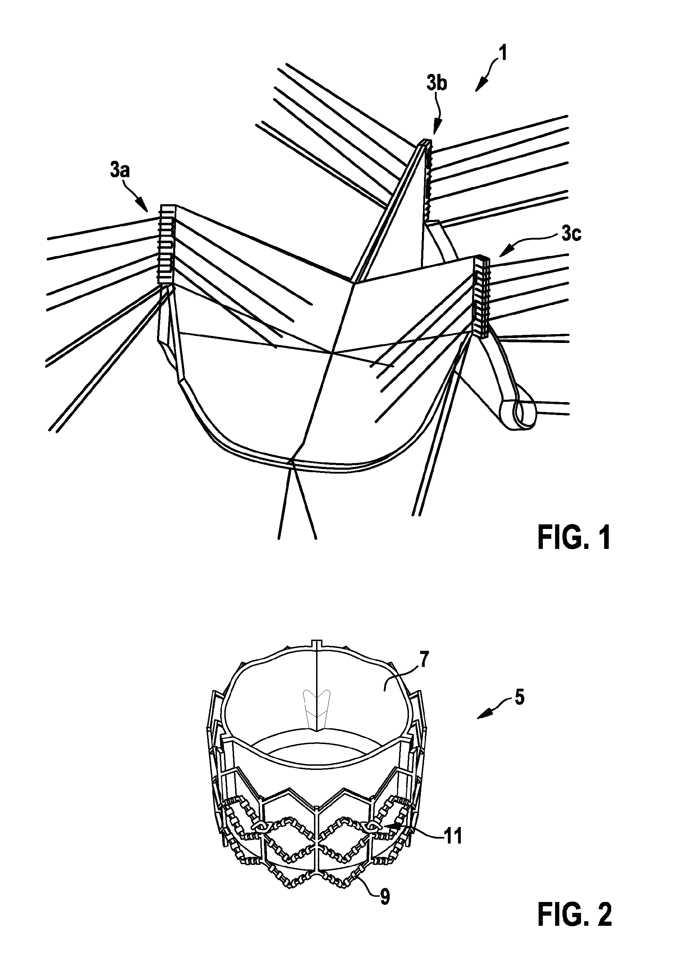

Fig. 1 zeigt eine Aortenklappen-Prothese 1, die etwa aus Schweine-, Pferde- oder Rinderperikardgewebe gefertigt sein kann, mit drei Nahtabschnitten 3a, 3b, 3c und mehreren (nicht separat bezeichneten) Referenznähten bzw. -stichen. Eine Ausführung der Nahtbereiche 3a, 3b, 3c mit einem röntgen-sichtbaren Metalldraht, beispielsweise aus Platin, macht die Nahtverläufe unter Röntgenkontrolle sichtbar und gibt dem Operateur damit einen Hinweis auf die Lage der Klappenebene.

Fig. 2 zeigt eine handelsübliche minimal invasiv implantierbare Herzklappenprothese 5, bei der die eigentliche Herzklappe 7 aus biologischem Material in einen Stent 9 eingefügt ist. Beispielhaft sind Knoten 11 aus röntgen-sichtbarem Material eingezeichnet, die sich bei Ausführung aus hinreichend dickem Metalldraht und ggfs. mit geeignetem Knotenaufbau im Röntgenbild wahrnehmbar vom Gittergerüst des Stent unterscheiden lassen und somit eine Beurteilung der Lage der Klappenebene erlauben.

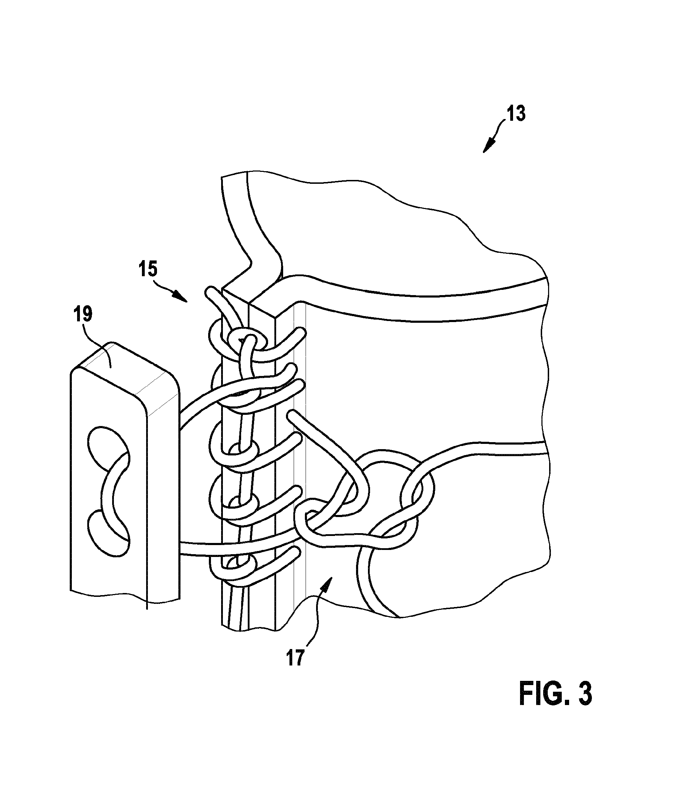

Fig. 3 zeigt als Detailansicht einer weiteren minimal invasiv implantierbaren Herzklappenprothese 13 einen Nahtabschnitt 15, mit dem Teile derselben miteinander vernäht sind, zusammen mit einem weiteren Naht-/Knotenabschnitt 17, an dem die eigentliche Herzklappe an einem Befestigungsabschnitt 19 eines zugehörigen Stents angebracht ist. Eine Ausführung des Nahtabschnitts 17 aus Metalldraht ermöglicht wiederum dessen Wahrnehmung im Röntgenbild und somit einen Rückschluss auf die Lage der Klappenebene.

Die Ausführung der Erfindung ist nicht auf die oben beschriebenen Beispiele und hervorgehobenen Aspekte beschränkt, sondern ebenso in einer Vielzahl von Abwandlungen möglich, die im Rahmen fachgemäßen Handelns liegen.

## Patentansprüche

1. Herzklappenprothese (1, 5; 13) aus biologischem Material oder Kunststoff, mit einer Naht (3a; 3b; 3c; 11; 17) aus einem röntgen-sichtbaren Metalldraht, deren Position die Klappenebene kennzeichnet.

2. Herzklappenprothese nach Anspruch 1, wobei der Metalldraht mindestens eines der Elemente Gold, Platin, Tantal und Wolfram aufweist, insbesondere aus Gold, Platin, Tantal oder Wolfram besteht oder mit mindestens einem der Elemente Gold, Platin, Tantal oder Wolfram beschichtet ist.

3. Herzklappenprothese nach einem der vorangehenden Ansprüche, wobei der Metalldraht einen Durchmesser im Bereich zwischen 20 und 200µm, insbesondere zwischen 50 und 100µm, hat.

4. Herzklappenprothese nach einem der vorangehenden Ansprüche, wobei die Naht aus dem Metalldraht einen Abschnitt einer aus einem anderen Nahtmaterial gefertigten Naht doppelt.

5. Herzklappenprothese nach einem der vorangehenden Ansprüche, wobei die Naht aus dem röntgen-sichtbaren Metalldraht als Doppel-, Kapp-, Overlock- oder sonstige optisch auffällige Naht ausgeführt ist.

6. Herzklappenprothese nach einem der vorangehenden Ansprüche, wobei die Klappensegel mit je einer Naht (3a; 3b; 3c) vernäht sind, wobei an jedem Klappensegel ein Nahtabschnitt aus dem röntgen-sichtbaren Metalldraht vorgesehen ist, derart, dass die drei Nahtabschnitte gemeinsam die Klappenebene kennzeichnen.

7. Herzklappenprothese nach einem der vorangehenden Ansprüche, wobei die Naht (3a; 3b; 3c) aus dem röntgen-sichtbaren Metalldraht derart platziert ist, dass ein vorbestimmtes Ende hiervon die Position der Klappenebene kennzeichnet.

8. Herzklappenprothese nach einem der vorangehenden Ansprüche, wobei die Naht aus dem röntgen-sichtbaren Metalldraht im Wesentlichen nur einen Knoten (11) umfasst.

9. Herzklappenprothese (5) nach einem der vorangehenden Ansprüche, eingenäht in einen Vaskularstent (9), wobei eine die Herzklappenprothese mit dem Vaskularstent verbindende Naht (17) korrespondierend zur Lage der Klappenebene angeordnet und aus dem röntgen-sichtbaren Metalldraht gebildet ist.

10. Herzklappenprothese nach einem der Ansprüche 1 - 8, eingenäht in einen Vaskularstent, wobei der Vaskularstent einen Nahtabschnitt aus dem röntgen-sichtbaren Metalldraht aufweist, deren Lage die Klappenebene kennzeichnet.
